# EUROPEAN PATENT APPLICATION

(11) **EP 4 529 940 A1**
(43) Date of publication of application: **02.04.2025**
(21) Application number: 24192007.3
(22) Date of filing: 31.07.2024
(51) Int. Cl.: A61M 16/04

(54) **ADJUSTABLE INTUBATION AID**

(30) Priority: 28.09.2023 TW 112137473
(71) Applicant: Hsieh, Ming-Feng, Kaohsiung City 813 (TW); Jiang, Jiaan-Tsuen, Taichung City 429 (TW)
(72) Inventor: Hsieh, Ming-Feng, Kaohsiung City 813 (TW); Jiang, Jiaan-Tsuen, Taichung City 429 (TW)
(74) Representative: Lang, Christian

(57) **Abstract**

An adjustable intubation aid is provided to solve the problem of inserting an endotracheal tube into a patent's trachea. The adjustable intubation aid includes a body (1), a control string (2), and a probe (3). The body (1) includes a first end (1a) and a second end (1b). An inner arcuate face (1c) is formed between the first end (1a) and the second end (1b) of the body (1). The second end (1b) of the body includes a head (12). The control string (2) includes a first end (2a) and a second end (2b). The first end (2a) of the control string (2) is connected to the inner arcuate face (1c) of the body (1). The second end (2b) of the control string (2) includes a pulling portion (21) having a slot (22) with an opening. The body (1) is inserted into the slot (22) via the opening. The probe (3) is made of soft material and envelops the first end (1a) of the body (1).

## Description

### BACKGROUND OF THE INVENTION

### 1. Field of the Invention

The present invention relates to an adjustable intubation aid and, more particularly, to an adjustable intubation aid enabling easy insertion of an endotracheal tube into a trachea of a patient.

### 2. Description of the Related Art

When proceeding with an intubation treatment on a patient, a medical person inserts an endotracheal tube from the oral cavity of a patient through the throat and the glottis of the patient into the deep of the trachea, and a ventilator is provided to assist in solving the patient's problems including breathing difficulty, faint breathing, or respiratory failure.

Since the endotracheal tube is generally made of soft medical material, during the intubation operation, the endotracheal tube made of soft medical material is inserted through the patient's throat via the patient's oral cavity into the deep of the trachea while avoiding the glottis. The intubation operation is extremely difficult due to the bend between the throat and the trachea. Thus, it often takes considerable time for the medical person to proceed with the intubation operation. Particularly, rapid completion of the intubation operation is relatively important when the patient requires urgent rescue.

Taiwan Patent Publication No. 201509450 discloses an endotracheal intubation assistance apparatus suitable for assisting in insertion of an endotracheal tube into the trachea of a patient. The endotracheal tube includes a tube body and a distal end. The endotracheal intubation assistance apparatus includes a movable tubular stylet, a graspable controller, and a viewing device. The movable tubular stylet is flexible and includes, along its axial direction, a head section, a body section, a tail section, and two slits extending through the body section and the tail section for dividing the tail section into first and second driven sheets. The viewing device includes an elongate body and a viewing head. The elongate body and the viewing head are slidable along the axial direction, extends through the graspable controller, and may extend outward through an inner hole of the head section of the movable tubular stylet. When the first and second driven sheets move relative to each other, the head section synchronously actuates the distal end of the endotracheal tube and the viewing head to swing.

The endotracheal intubation assistance apparatus disclosed in the above patent publication is complicated and difficult to operate. Particularly, the cleaning and disinfection after use are troublesome and inconvenient. Therefore, the above endotracheal intubation assistance apparatus cannot be widely used by medical persons to date.

Thus, a need exists for improvement to the conventional endotracheal intubation assistance apparatus to permit easy and rapid insertion of the endotracheal tube into the patient's trachea.

### SUMMARY OF THE INVENTION

To solve the above problem, the primary objective of the present invention is to provide an adjustable intubation aid which permits a worker to easily and rapidly insert an endotracheal tube into a patient's trachea.

The secondary objective of the present invention is to provide an adjustable intubation aid at a lost cost, such that the adjustable intubation aid may be discarded after use, which reduces the work of cleaning and disinfection after use, thereby reducing the workload of the worker while reducing cross contamination.

As used herein, the term "a", "an" or "one for describing the number of the elements and members of the present invention is used for convenience, provides the general meaning of the scope of the present invention, and should be interpreted to include one or at least one. Furthermore, unless explicitly indicated otherwise, the concept of a single component also includes the case of plural components.

An adjustable intubation aid according to the present invention includes a body, a control string, and a probe. The body includes a first end and a second end. An inner arcuate face is formed between the first end and the second end of the body. The second end of the body includes a head. The control string includes a first end and a second end. The first end of the control string is connected to the inner arcuate face of the body. The second end of the control string includes a pulling portion having a slot with an opening. The body is inserted into the slot via the opening. The probe is made of soft material and envelops the first end of the body.

Therefore, the adjustable intubation aid according to the present invention may extend through the hollow passageway of the endotracheal tube. Thus, during insertion of the insertion end of the endotracheal tube into the patient's throat and trachea, in a case that the insertion end of the endotracheal tube comes into contact with and is, thus, impeded by the wall of the throat, the worker may pull the pulling portion to cause bending and deformation of the probe toward the center of curvature of the arcuate inner face, such that the insertion end of the endotracheal tube is no longer impeded. This enables the endotracheal tube and its insertion end to easily and smoothly advance further, so as to move deeper into the trachea. Furthermore, the adjustable intubation aid according to the present invention may be discarded after use, which reduces the work of cleaning and disinfection, thereby reducing the workload of the worker while reducing cross contamination.

In an example, the body has a cross sectional area decreasing from the second end to the first end of the body. Therefore, the body may have a proper strength and the probe is apt to bend and deform.

In an example, the body is provided with a diameter difference portion adjacent to the first end through provision of a diameter difference. Therefore, the probe of the body may bend and deform more easily by the diameter difference portion.

In an example, the first end of the control string is connected to the inner arcuate face at a location between the diameter difference portion and the first end of the body. Therefore, the probe of the body may bend and deform more easily.

In an example, the control string and the body are integrally formed. Therefore, the control string and the body may be securely connected to avoid disengagement therebetween.

In an example, one of the control string and the body is formed first and placed into a mold for forming another of the control string and the body and is then enveloped by the other of the control string and the body after formation. Therefore, the control string and the body may be securely connected to avoid disengagement therebetween.

In an example, at least one of two walls of the slot includes a protruding portion to reduce a spacing between the two walls of the slot. Therefore, disengagement of the body via the opening may be prevented.

In an example, the body is formed first and placed into a mold for forming the probe, and the probe is then formed by injection molding to envelope the body. Therefore, the probe may envelope the first end of the body to avoid injury resulting from contact between the probe and the wall of the patient's throat.

In an example, the adjustable intubation aid further comprises an endotracheal tube having a hollow passageway. The endotracheal tube includes an insertion end and an operating end. Therefore, the adjustable intubation aid according to the present invention enables the endotracheal tube to easily and smoothly advance further, so as to move deeper into the deep of the trachea of the patient.

In an example, the probe extends through the hollow passageway of the endotracheal tube. Therefore, the adjustable intubation aid according to the present invention enables the endotracheal tube to easily and smoothly advance further, so as to move deeper into the deep of the trachea of the patient.

### BRIEF DESCRIPTION OF THE DRAWINGS

The present invention will become more fully understood from the detailed description given hereinafter and the accompanying drawings which are given by way of illustration only, and thus are not limitative of the present invention, and wherein:
FIG. 1 is a perspective view of an adjustable intubation aid of a preferred embodiment according to the present invention.
FIG. 2 is a front, cross-sectional view of an adjustable intubation aid of a preferred embodiment according to the present invention.
FIG. 3 is a diagrammatic view illustrating movement of the adjustable intubation aid of FIG. 2.
FIG. 4 is a diagrammatic view illustrating use of the adjustable intubation aid of a preferred embodiment according to the present invention.
FIG. 5 is another diagrammatic view illustrating use of the adjustable intubation aid of the preferred embodiment according to the present invention.

When the terms "front", "rear", "left", "right", "up", "down", "top", "bottom", "inner", "outer", "side", and similar terms are used herein, it should be understood that these terms have reference only to the structure shown in the drawings as it would appear to a person viewing the drawings and are utilized only to facilitate describing the invention, rather than restricting the invention.

### DETAILED DESCRIPTION OF THE INVENTION

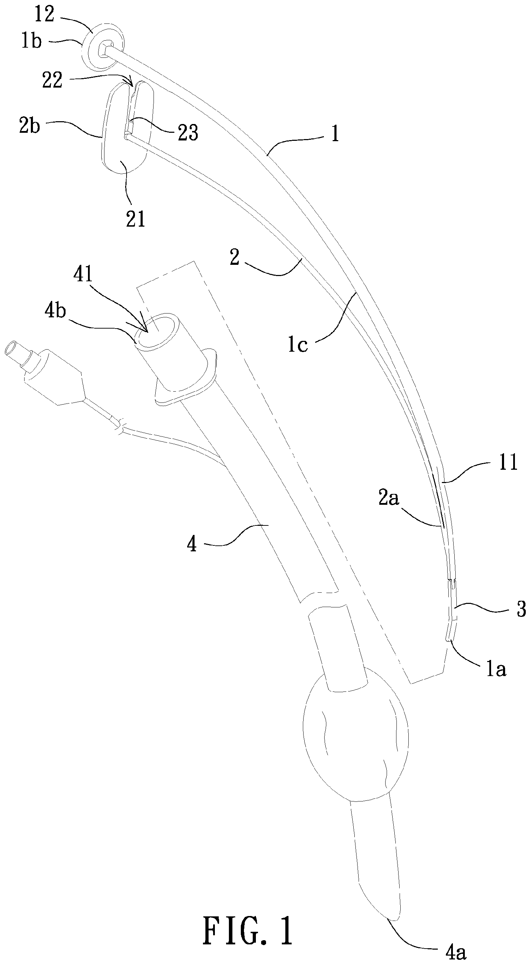

With reference to FIG. 1, an adjustable intubation aid of a preferred embodiment according to the present invention includes a body 1, a control string 2, and a probe 3. An end of the control string 2 is connected to the body 1. The probe envelops an end of the body 1.

The body 1 includes a first end 1a and a second end 1b. The body 1 includes an inner arcuate face 1c formed between the first end 1a and the second end 1b, such that the body 1 is flexible to permit bending and deformation. Namely, the first end 1a of the body 1 may bend and deform toward a center of curvature of the arcuate inner face 1c. The body 1 may have a cross sectional area decreasing from the second end 1b to the first end 1a of the body 1, such that the body has a proper strength and may bend and deform more easily. Preferably, the body 1 is provided with a diameter difference portion 11 adjacent to the first end 1a through provision of a diameter difference, such that the body 1 may have a greater amount of bending deformation due to the diameter difference portion 11. The second end 1b of the body 1 may further include a head 12 having a larger cross sectional area, such that a worker can easily abut his or her thumb against the second end 1b.

The control string 2 includes a first end 2a and a second end 2b. The first end 2a is connected to the arcuate inner face 1c of the body 1. Preferably, the first end 2a of the control string 2 is connected to the inner arcuate face 1c at a location between the diameter difference portion 11 and the first end 1a of the body 1. The connection between the control string 2 and the body 1 may be achieved through integral forming. Alternatively, one of the body 1 and the control string 2 is formed first and placed into a mold for forming another of the body 1 and the control string 2 and is then enveloped by the other of the body 1 and the control string 2 after formation. The present invention is not limited in this regard. The second end 2b of the control string 2 may include a pulling portion 21 having a slot 22 with an opening. The body 1 may be inserted into the slot 22 via the opening. Preferably, at least one of two walls of the slot 22 includes a protruding portion 23 to reduce a spacing between the two walls of the slot 22, thereby preventing disengagement of the body 1 via the opening.

The probe 3 envelops the first end 1a of the body 1. The probe 3 may be made of soft material, such as rubber, silicone, etc. to avoid injury resulting from contact between the probe 3 and the wall of the patient's throat. With regard to enveloping by the probe 3, the body 1 is formed first and placed into a mold for forming the probe 3, and the probe 3 is then formed by injection molding, such that the first end 1a of the body 1 may be enveloped by the probe 3.

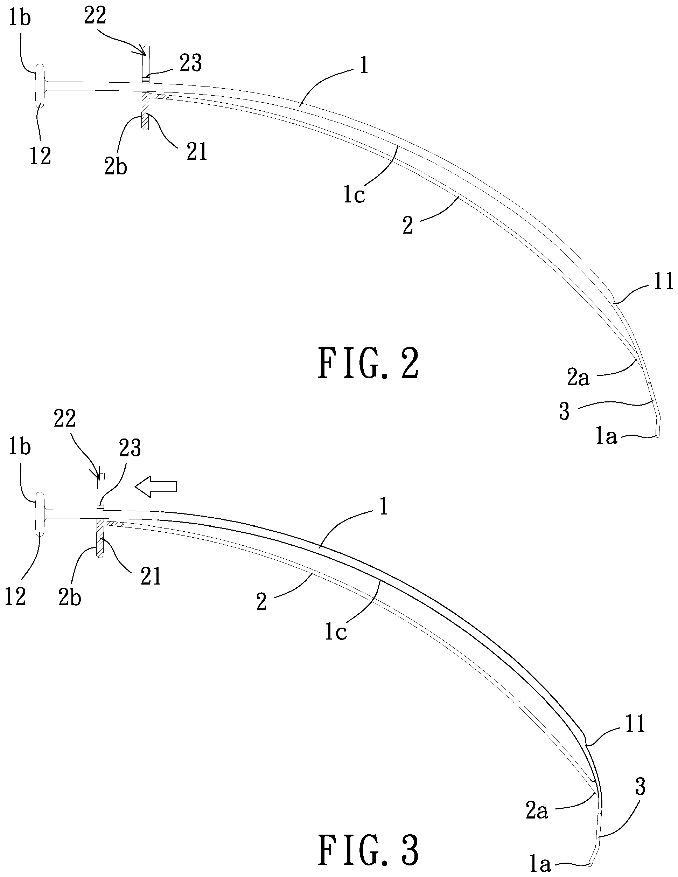

With reference to FIG. 2, the first end 2a of the control string 2 is connected to the arcuate inner face 1c of the body 1. The body 1 may be placed into the slot 22 via the opening, and the first end 1a of the body 1 protrudes to an outer side of the pulling portion 21.

With reference to FIG. 3, a worker may abut his or her thumb against the head 12 and may use the index finger and the middle finger to hold the second end 2b of the control string 2. Thus, the worker may pull the pulling portion 21 by the index finger and the middle finger to move the pulling portion 21 toward the head 12 of the body 1, as indicated by the arrow. Therefore, the first end 2a of the control string 2 may pull the first end 1a of the body 1 to enable the probe 3 on the first end 1a of the body 1 to bend and deform toward the center of curvature of the inner arcuate face 1c.

With reference to FIG. 1, the adjustable intubation aid according to the present invention may be used in an endotracheal tube 4 having a hollow passageway 41. The endotracheal tube 4 includes an insertion end 4a and an operating end 4b. The probe 3 of the adjustable intubation aid according to the present invention may extend through the hollow passageway 41 of the endotracheal tube 4 and may be used to pull the second end 2b of the control string 2 to enable bending of the insertion end 4a of the endotracheal tube 4. Therefore, the endotracheal tube 4 may be easily and rapidly inserted into the trachea of the patient.

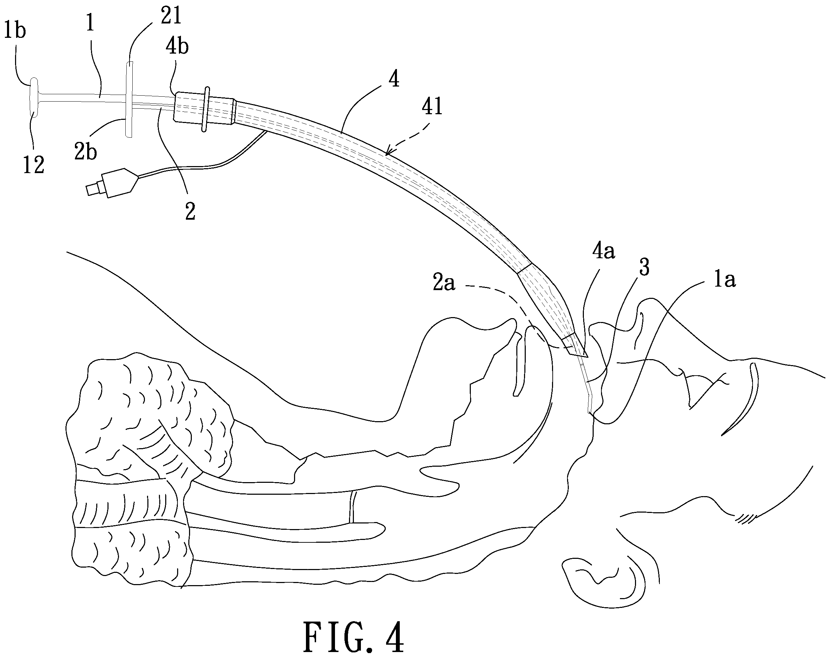

With reference to FIG. 4, the worker may extend the probe 3 of the adjustable intubation aid according to the present invention through the endotracheal tube 4, such that the probe 3 and the insertion end 4a of the endotracheal tube 4 may be placed into the patient's throat and inserted toward the throat and the trachea.

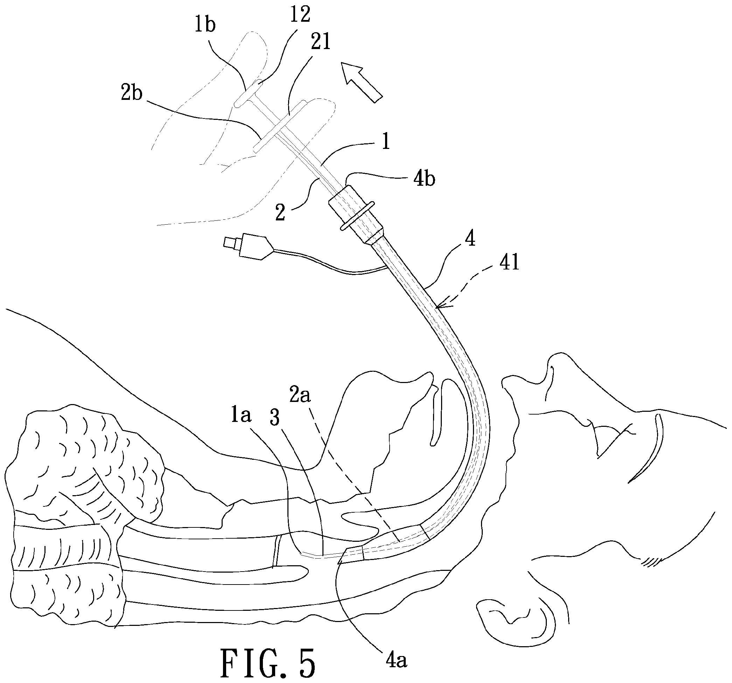

With reference to FIG. 5, when the probe 3 and the insertion end 4a of the endotracheal tube 4 come into contact with the wall of the throat and are, thus, impeded and therefore cannot be pushed forward smoothly, the worker may pull the pulling portion 21 by the index finger and the middle finger (as indicated by the arrow), thereby moving the pulling portion 21 toward the head 12 of the body 1. Thus, the first end 2a of the control string 2 may pull the body 1 to cause bending and deformation of the probe 3 toward the center of curvature of the arcuate inner face 1c. Furthermore, the bent, deformed probe 3 may also cause bending and deformation of the insertion end 4a of the endotracheal tube 4, such that the insertion end 4a no longer contacts with the wall of the throat and is, thus, not impeded. Therefore, the endotracheal tube 4 and its insertion end 4a may easily and smoothly advance further, so as to move deeper into the trachea of the patient.

In view of the foregoing, the adjustable intubation aid according to the present invention may extend through the hollow passageway of the endotracheal tube. Thus, during insertion of the insertion end of the endotracheal tube into the patient's throat and trachea, in a case that the insertion end of the endotracheal tube comes into contact with and is, thus, impeded by the wall of the throat, the worker may pull the pulling portion to cause bending and deformation of the probe toward the center of curvature of the arcuate inner face, such that the insertion end of the endotracheal tube is no longer impeded. This enables the endotracheal tube and its insertion end to easily and smoothly advance further, so as to move deeper into the trachea. Furthermore, the adjustable intubation aid according to the present invention may be discarded after use, which reduces the work of cleaning and disinfection, thereby reducing the workload of the worker while reducing cross contamination.

## Claims

1. An adjustable intubation aid **characterized in** comprising:
a body (1) including a first end (1a) and a second end (1b), wherein an inner arcuate face (1c) is formed between the first end (1a) and the second end (1b) of the body (1), and wherein the second end (1b) of the body (1) includes a head (12);
a control string (2) including a first end (2a) and a second end (2b), wherein the first end (2a) of the control string (2) is connected to the inner arcuate face (1c) of the body (1), wherein the second end (2b) of the control string (2) includes a pulling portion (21) having a slot (22) with an opening, and wherein the body (1) is inserted into the slot (22) via the opening; and
a probe (3) made of soft material and enveloping the first end (1a) of the body (1).

2. The adjustable intubation aid as claimed in claim 1, **characterized in that** the body (1) has a cross sectional area decreasing from the second end (1b) to the first end (1a) of the body (1).

3. The adjustable intubation aid as claimed in claim 1, **characterized in that** the body (1) is provided with a diameter difference portion (11) adjacent to the first end (1a) through provision of a diameter difference.

4. The adjustable intubation aid as claimed in claim 3, **characterized in that** the first end (2a) of the control string (2) is connected to the inner arcuate face (1c) at a location between the diameter difference portion (11) and the first end (1a) of the body (1).

5. The adjustable intubation aid as claimed in claim 1, **characterized in that** the control string (2) and the body (1) are integrally formed.

6. The adjustable intubation aid as claimed in claim 1, **characterized in that** one of the control string (2) and the body (1) is formed first and placed into a mold for forming another of the control string (2) and the body (1) and is then enveloped by the other of the control string (2) and the body (1) after formation.

7. The adjustable intubation aid as claimed in claim 1, **characterized in that** at least one of two walls of the slot (22) includes a protruding portion (23) to reduce a spacing between the two walls of the slot (22).

8. The adjustable intubation aid as claimed in claim 1, **characterized in that** the body (1) is formed first and placed into a mold for forming the probe (3), and the probe (3) is then formed by injection molding to envelope the body (1).

9. The adjustable intubation aid as claimed in claim 1, **characterized in** further comprising an endotracheal tube (4) having a hollow passageway (41), wherein the endotracheal tube (4) includes an insertion end (4a) and an operating end (4b).

10. The adjustable intubation aid as claimed in claim 9, **characterized in that** the probe (3) extends through the hollow passageway (41) of the endotracheal tube (4).
